# EUROPÄISCHE PATENTSCHRIFT

(11) **EP 3 520 839 B1**
(45) Veröffentlichungstag und Bekanntmachung des Hinweises auf die Patenterteilung: **15.04.2026**
(21) Anmeldenummer: 19154012.9
(22) Anmeldetag: 28.01.2019
(51) Int. Cl.: A61M 1/34, A61M 1/16, A61M 1/36

(54) **ÜBERWACHUNGSVORRICHTUNG UND VERFAHREN ZUR ÜBERWACHUNG EINER EXTRAKORPORALEN BLUTBEHANDLUNGSVORRICHTUNG**
MONITORING DEVICE AND METHOD FOR MONITORING AN EXTRACORPOREAL BLOOD TREATMENT DEVICE
DISPOSITIF DE SURVEILLANCE ET PROCÉDÉ DE SURVEILLANCE D'UN DISPOSITIF DE TRAITEMENT EXTRACORPOREL DU SANG

(30) Priorität: 31.01.2018 DE 102018102171
(43) Veröffentlichungstag der Anmeldung: 07.08.2019
(73) Patentinhaber: B. Braun Avitum AG, 34212 Melsungen (DE)
(72) Erfinder: STEGER, Jennifer, 34327 Körle (DE); BRUCHHÄUSER, Peter, 34121 Kassel (DE); NISSEN, Günter, 34212 Melsungen (DE)
(74) Vertreter: Winter, Brandl - Partnerschaft mbB

(56) Entgegenhaltungen:
- DE-A1- 102014 015 048
- DE-A1- 102015 016 271
- US-A1- 2009 292 236
- US-A1- 2017 364 649

## Beschreibung

Die Erfindung betrifft eine Überwachungsvorrichtung zur Überwachung einer extrakorporalen Blutbehandlungsvorrichtung, wie etwa einer Dialysemaschine, mit einem extrakorporalen Blutkreislauf, der eine arterielle Blutleitung mit einem arteriellen Patientenanschluss und/oder zumindest eine venöse Blutleitung mit einem venösen Patientenanschluss aufweist, und einem Dialysierflüssigkeitssystem, das eine Dialysierflüssigkeitszuleitung und eine Dialysierflüssigkeitsableitung aufweist. Daneben betrifft die Erfindung ein Verfahren zur Überwachung einer extrakorporalen Blutbehandlungsvorrichtung gemäß dem Oberbegriff des nebengeordneten Anspruchs.

### Hintergrund der Erfindung

Bei externen Blutbehandlungsvorrichtungen wie beispielsweise einer Dialysemaschine, ist es wichtig, eine einwandfreie Behandlung des Patienten gewährleisten zu können. Insbesondere müssen dabei Flüsse der Fluide kontrolliert und exakt gesteuert werden. Für eine Schaltung von Flüssen bzw. Flusswegen des Fluides, sei es etwa Blut oder eine Dialysierflüssigkeit, werden üblicherweise Ventile verwendet. Diese können beispielsweise Schlauchquetschventile/Schlauchklemmen oder auch in einer Leitung direkt eingebrachte Ventile sein. Hierbei ist es möglich, dass ein Ventil nicht korrekt schließt oder öffnet und insbesondere nicht vollständig schließt. Gründe hierfür können beispielsweise Partikel, Verkalkung oder Verschleiß sein, welche einen mechanischen Verschluss des Ventils behindern oder beispielsweise auch eine Pumpe beeinträchtigen können. Insbesondere bei Ventilen, welche essenziell für eine korrekte Bilanzierung des Fluidstroms sind, kann es zu einer Bilanzierungsabweichung (Ultrafiltrationsabweichung) kommen. Schließt ein Ventil bei einer Behandlung nicht korrekt, so kann dies die Behandlung gefährden und tiefgreifende Risiken für den zu behandelnden Patienten, im schlimmsten Fall sogar letale Folgen, mit sich bringen.

Bei beispielsweise einer Dialysetherapie mit einer Dialysemaschine ist es heutzutage üblich, vor jeder Dialysetherapie einen sogenannten Selbsttest der Dialysemaschine zu vollziehen. Mit Hilfe dieses Selbsttests kann unter anderem nachgewiesen werden, dass die Ventile sicher und korrekt schließen und sich betätigen lassen. Nach heutigem Stand der Technik werden diese Selbsttests periodisch, beispielsweise alle eins bis zwei Stunden wiederholt, um in diskreten Zeitabständen eine Überprüfung durchzuführen. Hierbei wird die Therapie kurzzeitig, meist einige Minuten, unterbrochen und anschließend fortgesetzt. Durch diesen Selbsttest kann also ein interner Defekt der Dialysemaschine festgestellt werden. Nachteilig ist allerdings, dass die periodischen Selbsttests jedoch nur in begrenzter Anzahl und mit Zeitabständen zueinander durchgeführt werden können. Zwischen den Selbsttests verbleibt eine Zeitperiode, welche nicht überprüft wird und in der rein statistisch gesehen vermutlich kein Defekt auftritt. Das bedeutet, dass in dieser Zeitperiode die Therapie und die korrekte Funktionsweise der Blutbehandlungsmaschine nicht überwacht werden kann. Zudem unterbrechen die Selbsttests eine Therapie und verlängern sowohl die Therapie- als auch die Dialysezeit.

Es werden neben den Selbsttests auch Flüssigkeits- bzw. Feuchtesensoren in den Blutbehandlungsvorrichtungen eingesetzt, um eine mögliche Leckage zu detektieren. DE 10 2014 100260 A1 offenbart beispielsweise ein System, bei dem eine Luftfeuchtigkeit von in das Gehäuse einströmender Luft mit einer Luftfeuchtigkeit der aus dem Gehäuse ausströmender Luft vergleicht. Nachteilig hierbei ist allerdings, dass das System bzw. die Flüssigkeitssensoren jedoch nur Leckagen in einer offenen Umgebung in dem Gehäuse detektieren können. Findet der Austritt des Fluid in einem in sich geschlossenen Volumen innerhalb des Gehäuses statt, so ändert sich auch nicht die Luftfeuchte und der Defekt kann nicht detektiert werden. Ebenso wenig kann eine in einem Kreislauf auftretende interne Leckage (als Spezialfall des geschlossenen Volumens), bei dem beispielsweise ein Ventil nicht korrekt schließt, durch ein solches System nicht detektiert werden.

Daneben ist es möglich, einen an ein Ventil angelegten elektrischen Strom oder eine angelegte elektrische Spannung zu überwachen, um zu analysieren, ob das Ventil ordnungsgemäß schaltet. Eine solche Überwachung ist allerdings auf ein Aufzeigen von nur einem elektrischen Fehler beschränkt und kann nicht beurteilen, ob eine Leckage durch beispielsweise eine mechanische Blockierung oder eines Verschleiß des Ventils vorliegt.

Weitere Defekte, welche in einer Blutbehandlungsmaschine und insbesondere in einer Dialysemaschine auftreten können, sind beispielsweise eine verschlissene Pumpe, dejustierte Drosseln, verstopfte Filter oder Abnutzungen sowie Produktfehler von technischen Komponenten.

Die DE 10 2009 024 864 A1 offenbart ein Verfahren sowie eine Vorrichtung zur Überwachung eines Flüssigkeitssystems einer extrakorporalen Blutbehandlungsvorrichtung. Hierbei wird der Druck in einer venösen Tropfkammer abzweigenden Belüftungsleitung gemessen und ausgewertet, wobei ein Ventil alternierend zumindest teilweise geöffnet und geschlossen wird. Das gemessene Drucksignal ist also Druckschwankungen unterworfen, wenn keine Störung vorliegt. Dabei wird ein Druck in einer Zuleitung gemessen und ein Trigger für eine Erzeugung eines Druckimpulses zusätzlich zu der Blutbehandlungsvorrichtung eingesetzt. Zudem ist es schwierig auf einer Dialysierflüssigkeitsseite ein Ventil zyklisch zu öffnen und zu schließen, um eine Druckänderung als Triggersignal zu generieren. Ein nicht korrekt schließendes Ventil lässt sich kaum detektieren.

Die WO 2007/006348 A1 und die DE 10 2009 060 668 A1 offenbaren ein Medikamentenzuführsystem für flüssige Medikamente und eine Überwachungsvorrichtung, welche eine Flussrate von gefördertem Blut überwacht. Der Einsatz von Zentrifugalpumpen ist bei letzterem Dokument notwendig, was eine Gestaltung einer Dialysemaschine beschränkt. Diese Systeme lassen sich ebenso schwierig auf die Dialysierflüssigkeitsseite übertragen, um festzustellen, ob dort ein Defekt vorliegt.

Die DE 10 2015 016 271 A1 offenbart ein System sowie ein Verfahren zur Erkennung eines Betriebszustandes oder eines Behandlungsverlaufs einer Blutbehandlung. Während einer Blutbehandlung wird ein Betriebsparameter einer Blutbehandlungsmaschine kontinuierlich überwacht und sein zeitabhängiger Verlauf eines Messsignales wird gespeichert. Weicht der Behandlungsverlauf von einem idealen oder komplikationsfreien Behandlungsverlauf ab, so erfolgt eine Anwendereingabe.

Die DE 10 2014 015 048 A1 offenbart ein Verfahren zur ereignisgesteuerten Grenzwertsetzung im Rahmen einer Überwachung einer Dialysemaschine. Hierbei werden Zustandsvariablen in einem Zustandsvektor gespeichert. Entsprechend dem Zustandsvektor zugehörige Messsignalvektoren werden mit Referenz-Zustandsvektoren verglichen und ein medizintechnisches Gerät wird mit Ziel-Grenzwerteinstellungen gesteuert und überwacht.

Die US 2009/0292236 A1 offenbart ein Verfahren zur Überwachung eines extrakorporalen Blutkreislaufs sowie eine Vorrichtung zur Erkennung einer Abweichung zwischen einem vorgegebenen Soll-Verlauf und dem tatsächlichen Verlauf.

### Zusammenfassung der Erfindung

Es sind die Aufgaben und Ziele der Erfindung, die Nachteile aus dem Stand der Technik zu beheben oder wenigstens zu mindern und insbesondere eine Überwachungsvorrichtung und ein Verfahren zur Verfügung zu stellen, die/das Defekte mit minimalem Zusatzaufwand an unterschiedlichsten Stellen der Blutbehandlungsvorrichtung mit großer Aussagekraft aufzuspüren vermag und eine sichere, zuverlässige, kostengünstige sowie einfache Überwachung einer extrakorporalen Blutbehandlungsvorrichtung gewährleistet sowie eine Zeit einer Dialysetherapie verkürzt und zudem noch eine Feinjustierung von Überwachungsparametern zulässt. Zudem soll eine kontinuierliche Überwachung während der Behandlung möglich sein.

Die Aufgaben und Ziele werden hinsichtlich einer gattungsgemäßen Überwachungsvorrichtung erfindungsgemäß durch den Gegenstand der unabhängigen Ansprüche gelöst. Die Erfindung beruht also auf der Erkenntnis, dass die Blutbehandlungsvorrichtung, ohne weitere notwendige Veränderungen der Blutbehandlungsvorrichtung, ohnehin laufend genügend Messwerte/Parameter bereitstellt, die eine ausreichende Aussagekraft über die Funktionszuverlässigkeit der Blutbehandlungsvorrichtung insgesamt bzw. von ausgewählten Komponenten oder Abschnitten haben.

Die Überwachungsvorrichtung gemäß Anspruch 1 ist konfiguriert/dafür angepasst, (zumindest) einen durch und während des Betriebs der extrakorporalen Blutbehandlungsvorrichtung auftretenden und erfassten Messwert/Messsignal/Zustandsmesswert/Parameter, welcher zur Überwachung der Blutbehandlungsvorrichtung mit hinreichender Aussagekraft geeignet ist, zu selektieren und abzugreifen. In anderen Worten steht die Überwachungsvorrichtung also mit zumindest einem Sensor/Detektor der Blutbehandlungsvorrichtung, welcher Messwerte/Zustandsparameter der extrakorporalen Blutbehandlungsvorrichtung erfasst, in Verbindung oder ist mit diesem koppelbar.

Die Überwachungsvorrichtung ist demgemäß dafür angepasst, einen Messwert der extrakorporalen Blutbehandlungsvorrichtung abzugreifen, welcher für den Betrieb der extrakorporalen Blutbehandlungsvorrichtung mit zumindest einem Sensor der Blutbehandlungsvorrichtung erfasst wird. Hierbei werden keine zusätzlichen Komponenten, wie etwa ein Trigger zur Erzeugung periodischer Druckimpulse, benötigt, sondern es wird auf bereits vorhandene Messwerte, welche bei dem Betrieb der Blutbehandlungsvorrichtung bzw. während der Behandlung erfasst werden, zurückgegriffen. Insbesondere werden nur "Kern-Messwerte" verwendet". "Kern-Messwerte" bedeutet in diesem Fall, dass diese Messwerte für eine ordnungsgemäße Behandlung ohnehin mittels der Blutbehandlungsvorrichtung erhoben werden müssen. Es wird somit soweit wie möglich auf einen bereits vorhandenen "Datenpool" der Blutbehandlungsvorrichtung und auf vorhandene Sensoren zurückgegriffen und durch entsprechende Algorithmen und Auswertungsmethoden eine Fehlfunktion bzw. ein Defekt der Vorrichtung oder von einer oder mehrerer ausgewählter Komponenten der Vorrichtung detektiert. Dadurch, dass für mehrere unterschiedliche Messwerte unterschiedliche Soll-Verläufe hinterlegt sind, erhöht sich eine Aussagekraft der Überwachungsvorrichtung. Auch können Korrelationen der Messwerte untereinander berücksichtigt werden. Die Überwachungsvorrichtung kann über die Auswertung von "Kern-Messwerten" an unterschiedlichsten Stellen der Blutbehandlungsvorrichtung Defekte detektieren. Aufgrund einer über die Behandlungsdauer quasi kontinuierlichen Erfassung der Messwerte kann auch auf ein verzögernd schaltendes Ventil geschlossen werden. Schließt beispielsweise ein Ventil nicht korrekt, so ändern sich die Druckverläufe charakteristisch. Dies lässt sich insbesondere bei Ventilen feststellen, welche zyklisch geöffnet und geschlossen werden. Der Überwachungsvorrichtung bzw. dem Speicher der Überwachungsvorrichtung können für den Vergleich und die Bestimmung vorzugsweise auch Methoden der Mustererkennung hinterlegt sein.

Dementsprechend kann die Überwachungsvorrichtung gemäß Anspruch 1 mithilfe von erfassten Messwerten, wie beispielsweise Druck, Temperatur, Flussrate, Leitfähigkeit und/oder Membranstellung, während einer laufenden Behandlung bzw. während eines Betriebes der Blutbehandlungsvorrichtung ohne Unterbrechung der Behandlung auf einen bzw. ggf. auch auf mehrere Defekte schließen . Durch die Hinterlegung eines Soll-Verlaufs in dem Speicher der Überwachungsvorrichtung, kann eine definierte (ideale) Vorgabe gespeichert werden, bei der gesichert ist, dass die Behandlung korrekt verläuft. Mithilfe der Überwachungsvorrichtung kann kontinuierlich, sicher und zuverlässig auf einen Defekt in der Blutbehandlungsvorrichtung geschlossen werden, und insbesondere interne Leckagen effizient erkannt werden. Die Soll-Verläufe können beispielsweise durch experimentelle Messungen im Vorfeld oder über einen computergestützten, simulierten Verlauf definiert werden.

Gemäß Erfindung sind in dem Speicher der Überwachungsvorrichtung (neben dem Soll-Verlauf des Messwertes) also ein oder mehrere Fehler-Verläufe des Messwertes hinterlegt, wobei die Überwachungsvorrichtung bestimmt, dass ein Defekt vorliegt, wenn zumindest abschnittsweise der Ist-Verlauf des erfassten Messwertes von dem Soll-Verlauf des hinterlegten Messwertes über eine definierte Toleranz hinaus abweicht und wenn zumindest abschnittsweise der Ist-Verlauf des erfassten Messwertes innerhalb einer definierten zweiten Toleranz mit dem hinterlegten Fehler-Verlauf des Messwertes übereinstimmt. Hierbei können insbesondere charakteristische Fehler-Verläufe von beispielsweise eines nicht korrekt schließenden Ventils, einer verschlissenen Pumpe mit verminderter Pumpleistung, einer dejustierten Drossel mit entsprechenden Folgeerscheinungen oder eines verstopften Filters als Referenz in dem Speicher hinterlegt werden. Diese Fehler-Verläufe können im Vorfeld experimentell oder theoretisch bestimmt werden. Infolgedessen kann, wenn nur ein oder eine begrenzte Anzahl an Fehlern auftritt, durch einen Vergleich des Ist-Verlaufs mit dem entsprechenden Fehler-Verlauf auch direkt auf eine Fehlerursache, wie beispielsweise einen abgeknickten Schlauch oder eine Verstopfung, geschlossen werden, so dass eine Nachvollziehbarkeit und eine Reparatur erleichtert wird. Insbesondere kann die Überwachungsvorrichtung die Blutbehandlungsvorrichtung anleiten, nach Bestimmung eines Defekts, einen ausführlichen Selbsttest durchzuführen und den Defekt zu analysieren.

Vorteilhafte Ausführungsformen sind in den Unteransprüchen beansprucht und werden nachfolgend erläutert.

In einer bevorzugten Variante kann die Überwachungsvorrichtung die Messwerte während eines Betriebs der extrakorporalen Blutbehandlungsvorrichtung ohne Zeitverzug zeitaktuell erfassen, dass bedeutet, dass lediglich eine physikalisch auftretende Signallaufzeit mit zugehöriger Signalverarbeitung, aber keine weitere (gewollte und vermeidbare) Verzögerung auftritt. Hierdurch kann eine Art "online" bzw. zeitaktuelle Überwachungsvorrichtung geschaffen werden, welche es einem Anwender bzw. einem Bedienpersonal ermöglicht, bei einem von der Überwachungsvorrichtung bestimmten Defekt bzw. einer Fehlfunktion von sowohl der Blutbehandlungsvorrichtung als auch einer Komponente sofort einzugreifen bzw. die Überwachungsvorrichtung dazu befähigt, den Defekt sofort, sprich ohne Zeitverzug, festzustellen und notwendige Maßnahmen, wie eine Unterbrechung der Behandlung beispielsweise durch Schließen eines Ventils oder andere adäquate Maßnahmen einzuleiten, so dass eine Gefährdung des Patienten ausgeschlossen oder zumindest minimiert wird. Die Überwachungsvorrichtung kann mithin "online", also während der Behandlung, ohne Unterbrechung der Therapie und ohne zusätzliche Tests, wie einem Selbsttest, welche nur zur Bestimmung bestimmter Defekte geeignet ist, einen Defekt bzw. eine Fehlfunktion einer Komponente der Blutbehandlungsvorrichtung und damit der Blutbehandlungsvorrichtung bestimmen. "Online" definiert dabei ein Verfahren, welches während der Therapie durchgeführt werden kann, ohne die Therapie zu unterbrechen und ohne dedizierte nur für die Detektion eines Fehlers notwendige Abläufe durchführen zu müssen. Das bedeutet, dass ein "Online"-Verfahren eine Detektion eines Defekts allein aufgrund der durch die Therapie verursachten Signalverläufe erlaubt. Unter dem Begriff Defekt wird ebenso auch eine Fehlfunktion verstanden.

Insbesondere weist die Überwachungsvorrichtung eine Schnittstelle auf, mit der die Überwachungsvorrichtung mit der extrakorporalen Blutbehandlungsvorrichtung zumindest datentechnisch verbunden bzw. verbindbar ist. Insbesondere kann diese Schnittstelle drahtlos/kabellos in Form von beispielsweise Bluetooth^{®} oder WLAN vorliegen. In dem Falle, dass die Überwachungsvorrichtung bestimmt, dass ein Defekt der Blutbehandlungsvorrichtung vorliegt, kann die Überwachungsvorrichtung über die Schnittstelle einen entsprechenden Steuer-Befehl und/oder einen entsprechenden Fehlercode an die Blutbehandlungsvorrichtung senden, damit diese adäquate Maßnahmen zum Schutz des Patienten einleitet.

In einer bevorzugten Ausführungsform kann die Überwachungsvorrichtung eine Spannung und/oder einen Strom und/oder eine Drehzahl einer in der extrakorporalen Blutbehandlungsvorrichtung in dem extrakorporalen Blutkreislauf zum Fördern von Blut und/oder in dem Dialysierflüssigkeitssystem zum Fördern von Dialysierflüssigkeit angeordneten Pumpe als erfassten Messwert abgreifen. Hierbei kann bestimmt werden, ob ein Defekt vorliegt, da beispielsweise über eine Drehzahl der Pumpe festgestellt werden kann, ob ein nachfolgendes oder ein vorangegangenes Ventil korrekt (komplett) geschlossen ist oder entgegen der Schaltung doch einen Durchfluss erlaubt. Zusätzlich oder alternativ kann die Überwachungsvorrichtung einen Druck und/oder eine Temperatur und/oder einen Durchfluss und/oder eine Leitfähigkeit und/oder eine Membranposition als erfassten Messwert abgreifen.

In einer bevorzugten Ausführungsform, kann die Überwachungsvorrichtung nach einem Startsignal für eine vorbestimmte Detektions-Zeit den Ist-Verlauf des Messwertes bzw. der jeweiligen Messwerte detektieren und diesen detektierten Ist-Verlauf jeweils als den in dem Speicher der Überwachungsvorrichtung hinterlegten Soll-Verlauf des entsprechenden Messwerts festsetzen. Auf diese Weise kann für jede extrakorporale Blutbehandlungsvorrichtung individuell, beispielsweise nach Prüfung, dass die Blutbehandlungsvorrichtung auch ordnungsgemäß funktioniert, ein Ist-Verlauf für jeden relevanten Messwert aufgezeichnet werden (eine Art geprüfter, korrekter Verlauf), welcher dann anschließend als Soll-Verlauf des entsprechenden Messwerts in dem Speicher gespeichert wird. Hierbei kann die Überwachungsvorrichtung an unterschiedliche Blutbehandlungsvorrichtungen angeschlossen/verbunden und über die Aufzeichnung auch entsprechend kalibriert werden. Eine detaillierte Vorlage und Definition eines Soll-Verlaufs für jeden relevanten Messwert bereits während einer Produktion der Blutbehandlungsvorrichtung (für jedes einzelne Gerät, mit Feinkalibrierung) ist nicht notwendig. Auch lässt sich zeitaktuell, nach Prüfung der korrekten Funktionsweise der Blutbehandlungsmaschine, der Soll-Verlauf erfassen, so dass externe Einflüsse, welche im Laufe der Produktion und eines Betriebs natürlicherweise vorkommen, auf eine Veränderung der erfassten Messwerte stark reduziert werden.

Vorzugsweise kann als Kriterium für die definierte erste und/ oder zweite Toleranz eine Steigung und/oder eine absolute Messwertabweichung und/oder eine Standardabweichung zwischen dem Ist-Verlauf und dem Soll-Verlauf und/oder zwischen dem Ist-Verlauf und dem Fehler-Verlauf des Messwertes herangezogen werden. Ein theoretisch oder experimentell festgelegter Soll-Verlauf bzw. Fehler-Verlauf wird in der Realität nicht 100% durch den Ist-Verlauf getroffen, sondern vielmehr wird sich der Ist-Verlauf in einem Bereich, bzw. anschaulich gesehen, innerhalb eines gewissen Schlauchs/Korridor als Vertrauensbereich um den Soll-Verlauf bzw. den Fehler-Verlauf bewegen. Liegt der Ist-Verlauf innerhalb des Vertrauensbereichs um den Soll-Verlauf, so bestimmt die Überwachungsvorrichtung, dass kein Defekt vorliegt. Die Definition bzw. Ausgestaltung dieses Vertrauensbereichs bzw. der festgelegten Grenzen werden durch die obigen Kriterien erreicht. Insbesondere können auch alle drei Kriterien, nämlich eine Steigung, eine absolute Messwertabweichung oder eine Standardabweichung gefordert sein, so dass die Abweichung von nur einem Kriterium reicht. Ebenso kann auch gefordert sein, dass nur dann reicht, wenn die Steigung und die absolute Messwertabweichung und die Standardabweichung des Ist-Verlaufs zu dem Soll-Verlauf bzw. dem Fehler-Verlauf abweichen. Hierdurch wird einem Fehlalarm vorgebeugt, da alle drei Kriterien erfüllt sein müssen. Vorzugsweise kann alternativ oder zusätzlich ein Approximationsfehler bzw. eine Messwertabweichung herangezogen werden.

Zusätzlich oder alternativ kann in einer bevorzugten Variante für die definierte erste und/ oder zweite Toleranz ein Minimum und/oder ein Maximum über einen definierte Zeitraum des Ist-Verlaufs des erfassten Messwerts gegenüber dem hinterlegten Soll-Verlauf und/oder dem hinterlegten Fehler-Verlauf des Messwerts als Kriterium herangezogen werden. Hierbei kann (Zeit-)abschnittsweise in einer Art Stufenform eine Art Begrenzung des Soll-Verlaufs, in dem sich der Ist-Verlauf bewegen soll, nach oben und/oder unten vorgenommen werden. Dieser Zeitabschnitt kann beispielsweise ein Bilanzkammerzyklus oder ein Spülzyklus sein. Vorzugsweise kann auch eine (absolute) Differenz zwischen erstem und letztem erfassten Messwerte in einem definierten Zeitraum als Kriterium herangezogen werden.

In einer bevorzugten und ggf. unabhängig zu beanspruchenden Ausführungsform, kann die Überwachungsvorrichtung zumindest zwei (sprich mehrere) Messwerte erfassen und abgreifen. Die Überwachungsvorrichtung kann ein Verhalten und/oder eine Korrelation der Messwerte zueinander bei dem Vergleich der Verläufe und der Bestimmung, dass ein Defekt vorliegt, heranziehen. Die Überwachungsvorrichtung kann also nicht nur die Messwerte einzeln und separat voneinander betrachten, sondern bei einer Auswertung der zumindest zwei Messwerte eine Korrelation der Messwerte untereinander berücksichtigen und für die Auswertung bzw. Bestimmung, ob ein Defekt vorliegt, heranziehen. Beispielsweise kann ein Druck und eine Temperatur erfasst werden. Wenn der Druck sowie die Temperatur einen entsprechenden Verlauf aufweisen, der miteinander korreliert, kann die Überwachungsvorrichtung bestimmen, dass ein Defekt vorliegt.

Gemäß einem weiteren Aspekt der Erfindung, kann bei Feststellung und Bestimmung eines Defekts durch die Überwachungsvorrichtung ein akustisches, visuelles und/oder taktiles (Alarm-)Signal durch eine Ausgabeeinheit ausgegeben werden. Die Störung kann damit sowohl akustisch, visuell als auch taktil einem Bediener der Blutbehandlungsvorrichtung mitgeteilt werden und bei einer Störung kann ein Eingriff in die Steuerung der Blutbehandlungsvorrichtung vorgenommen werden. Beispielsweise könnte durch das Alarmsignal der Anwender und der Patient informiert werden, und der Anwender kann eine Blutpumpe anhalten und eine venöse Schlauchklemme schließen bzw. wenn dies bereits automatisch passiert ist, darüber informiert werden, dass ein Defekt vorliegt.

Die Aufgabe und die Ziele der Erfindung werden auch durch ein Verfahren gemäß Anspruch 11 gelöst. Das Verfahren vergleicht, ähnlich zu der Überwachungsvorrichtung, den Ist-Verlauf des abgegriffenen, erfassten Messwerts mit dem zugehörigen Soll-Verlauf und dem Fehler-Verlauf des Messwerts und bestimmt demgemäß, ob ein Defekt vorliegt. Die Erfindung beruht hinsichtlich des Verfahrens, gleich wie bei der Überwachungsvorrichtung, auf der Erkenntnis, dass die Blutbehandlungsvorrichtung, ohne weitere notwendige Veränderungen, ohnehin laufend genügend Messwerte/Parameter bereitstellt, die eine ausreichende Aussagekraft über die Funktionszuverlässigkeit der Blutbehandlungsvorrichtung insgesamt bzw. von ausgewählten Komponenten oder Abschnitten haben.

In einer bevorzugten Ausführungsform/Variante des Verfahrens kann der Schritt des Vergleichens folgende Schritte umfassen: Bilden der Standardabweichung zwischen dem Ist-Verlauf des Messwertes mit dem Soll-Verlauf und/oder dem Fehler-Verlauf des Messwertes; und Vergleichen des Werts der gebildeten Standardabweichung mit einer hinterlegten Soll-Standardabweichung. Das Kriterium einer Standardabweichung bzw. Stichprobenabweichung für die erste und/ oder zweiteToleranz ist eine gute Wahl einer zu implementierende Funktion, da die Standardabweichung eine gewisse Fehlertoleranz über die Zeit zulässt und ein einzelner Wert, nämlich die Soll-Standardabweichung, ausreicht, um zu bestimmen, ob die Verläufe sich entsprechen. Hierbei kann für eine gesamte Zeit einer Behandlung oder für bestimmte (Zeit-)Abschnitte jeweils eine zugehörige Soll-Standardabweichung definiert werden.

### Kurzbeschreibung der Figuren

Die Erfindung wird nachfolgend anhand bevorzugter Beispiele mit Hilfe von Figuren näher erläutert. Es zeigen:
Fig. 1 zeigt in einer stark vereinfachten schematischen Ansicht ein erstes Beispiel einer Überwachungsvorrichtung mit einer extrakorporalen Blutbehandlungsvorrichtung, wobei diese Blutbehandlungsvorrichtung nicht gemäß Erfindung ist,
Fig. 2 einen Graph eines Vergleichs der Überwachungsvorrichtung bei dem ein Ist-Verlauf, innerhalb einer Toleranz mit einem Soll-Verlauf verglichen wird,
Fig. 3 einen Graph eines Vergleichs der Überwachungsvorrichtung bei dem der Ist-Verlauf nicht dem Soll-Verlauf entspricht,
Fig. 4 einen Graph eines Vergleichs der Überwachungsvorrichtung bei dem ein Ist-Verlauf, innerhalb einer Toleranz mit einem Fehler-Verlauf verglichen wird,
Fig. 5 einen Graph eines Vergleichs der Überwachungsvorrichtung bei dem der Ist-Verlauf nicht dem Fehler-Verlauf entspricht,
Fig. 6 einen Graphen mit einer beispielhaften Gegenüberstellung eines Soll-Verlaufs und eines Fehlerverlaufs, und
Fig. 7 ein Flussdiagramm eines Verfahrens zur Überwachung einer extrakorporalen Blutbehandlungsvorrichtung. DieseVerfahren ist allerdings nicht gemäß Erfindung.

Die Figuren sind schematischer Natur und dienen lediglich dem Verständnis der Erfindung. Gleiche Elemente sind mit denselben Bezugszeichen versehen. Die Merkmale der verschiedenen Beispiele können untereinander ausgetauscht werden.

### Detaillierte Beschreibung bevorzugter Ausführungsformen

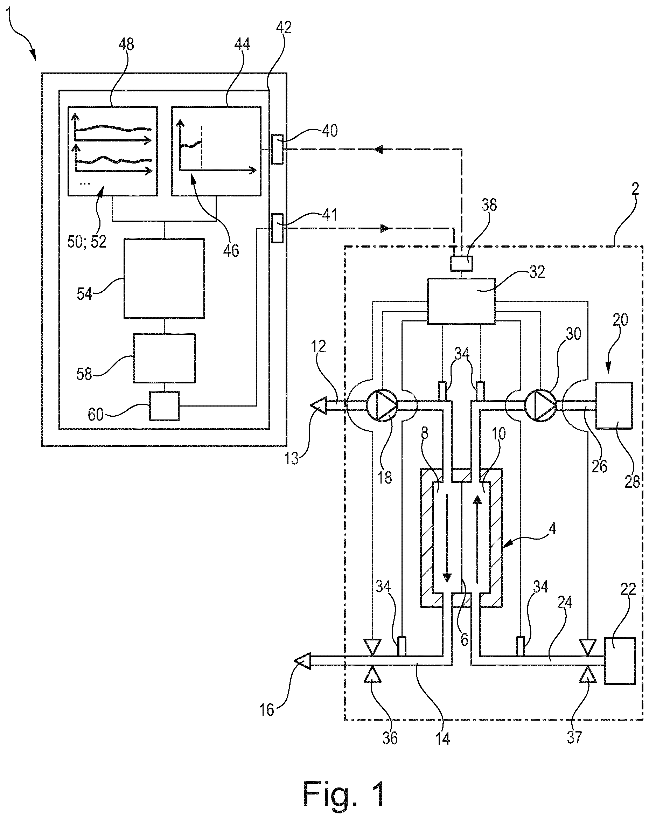

Figur 1 zeigt eine bevorzugte Ausführungsform einer erfindungsgemäßen Überwachungsvorrichtung 1. Die Überwachungsvorrichtung 1 dient zur Überwachung einer extrakorporalen Blutbehandlungsvorrichtung, insbesondere Hämo(dia)filtrationsvorrichtung, in Art einer Dialysemaschine 2 zur Blutreinigung bzw. Filtration von Blut. Figur 1 zeigt nur die wesentlichen Komponenten der Blutbehandlungsvorrichtung in schematischer Darstellung. Die Dialysemaschine 2 weist dabei einen Dialysator 4 oder Filter auf, welcher durch eine semipermeable Membran 6 in eine erste Kammer (Blutkammer) 8 und in eine zweite Kammer (Dialysierflüssigkeitskammer) 10 unterteilt ist.

Von einem Patienten (nicht dargestellt) führt von einer arteriellen Punktionskanüle 13 (als arterieller Patientenanschluss) aus eine arterielle Blutleitung 12 in Form eines flexiblen, elastischen Schlauchs zu der ersten Kammer 8 des Dialysators 4. Die Fließrichtung des Blutes ist dabei durch einen Pfeil in Figur 1 gekennzeichnet. Von dem Auslass der ersten Kammer 8 führt eine venöse Blutleitung 14 wieder zu dem Patienten zurück. Die venöse Blutleitung 14 in Form eines flexiblen, elastischen Schlauchs, wird mit einer venösen Punktionskanüle 16 an einem Shunt oder einer Fistel an dem Patienten angeschlossen. Zum Fördern des Blutes des Patienten ist in der arteriellen Blutleitung 12 eine peristaltische Blutpumpe 18 angeordnet. Außerdem ist in der venösen Blutkammer ein Blasenfänger angeordnet (nicht dargestellt), um vor der Rückführung Luft aus dem geförderten Blut zu entfernen.

Ein Dialysierflüssigkeitssystem 20 der Blutbehandlungsvorrichtung umfasst eine Dialysierflüssigkeitsquelle 22, an die eine Dialysierflüssigkeitszuleitung 24 angeschlossen ist, welche in die zweite Kammer 10 des Dialysators 4 führt. Von dem Auslass der zweiten Kammer 10 geht eine Dialysierflüssigkeitsableitung 26 ab, welche zu einem Dialysierflüssigkeitsabfluss 28 führt. Die Dialysierflüssigkeit wird in dem Dialysierflüssigkeitssystem 20 mit mindestens einer Dialysierflüssigkeitspumpe 30, wie beispielsweise einer Peristaltik-Pumpe oder einer Zentrifugalpumpe, gefördert.

Die Steuerung der Dialysemaschine 2 erfolgt durch eine elektronische Steuereinheit (ECU) 32. Die ECU 32 steuert zum einen unter anderem die Blutpumpe 18 sowie die Dialysierflüssigkeitspumpe 30 und zum anderen erfasst die ECU 32 Messwerte der Blutbehandlungsvorrichtung. Hierfür ist an jeder der Leitungen, nämlich an der arteriellen Blutleitung 12, der venösen Blutleitung 14 als auch der Dialysierflüssigkeitszuleitung 24 und der Dialysierflüssigkeitsableitung 26, jeweils ein Sensor 34 angebracht. Der Sensor 34 ist immer der gleiche Sensor 34 und erfasst dabei einen Druck, eine Temperatur sowie eine Flussrate der jeweiligen Leitung 12, 14, 24, 26. Natürlich können stattdessen auch unterschiedliche Sensoren mit unterschiedlichen Funktionsweisen, je nach Verwendung, angeordnet werden. Natürlich könnte beispielsweise auch nur lediglich ein Sensor 34 an der venösen Blutleitung 14, welcher auch nur den Druck als Messwert erfasst, angeordnet sein. Wichtig ist nur, dass die Dialysemaschine 2 zumindest einen Sensor 34 aufweist, um Messwerte für die ECU 32 zu erfassen.

Die Sensoren 34 leiten die Daten/Informationen der Messsignale bzw. Messwerte kabelgebunden oder kabellos an die ECU 32 weiter. Zur Aktuierung bzw. Steuerung des blutseitigen Kreislaufs, weist die Dialysemaschine 2 unter anderem ein Ventil in Form einer elektromagnetischen Schlauchklemme 36 auf, welche den Schlauch der venösen Blutleitung 14 quetschend abdichtet und damit den Blutstrom stoppt. Bei geschlossener venöser Schlauchklemme ist die Flüssigkeitsströmung im extrakorporalen Blutkreislauf also unterbrochen. Auf Seiten des Dialysierflüssigkeitssystems 20 weist die Dialysemaschine 2 ebenfalls ein Ventil 37 auf, welches den Fluss durch die Dialysierflüssigkeitszuleitung 24 steuern sowie auch stoppen kann. Die Dialysemaschine 2 weist überdies eine Schnittstelle 38 auf, mit welcher die Dialysemaschine 2 datentechnisch mit der Überwachungsvorrichtung 1 verbunden ist. Dem Verständnis und der Anschauung halber ist in Figur 1 der Datenfluss der Verbindung getrennt in Datenausgang und Dateneingang aufgeteilt. Schließt beispielsweise das Ventil 37 nicht korrekt, so kann dies die Überwachungsvorrichtung 1 detektieren.

In der extrakorporalen Blutbehandlungsvorrichtung 2 sind ebenso Bilanzkammern zur volumetrischen Bilanzierung von Flüssigkeiten vorgesehen. Bei der Bilanzkammer handelt es sich um eine Kammer, die durch eine elastische Membran in zwei Kompartimente geteilt ist. Je nachdem, wie viel Flüssigkeit sich jeweils in den beiden Kompartimenten befindet, wird die Membran entweder zu dem einen oder zu dem anderen Kompartiment hin ausgelenkt. Mithilfe eines Positionssensors kann die extrakorporale Blutbehandlungsvorrichtung 2 die Position der Membran messen. Zusätzlich sind in der extrakorporalen Blutbehandlungsvorrichtung 2 Leitfähigkeitszellen vorgesehen, um Dialysekonzentrate in der richtigen Konzentration einer Dialysierflüssigkeit hinzu zu dosieren. Beim Betrieb der extrakorporalen Blutbehandlungsvorrichtung 2 werden die entsprechenden Signalverläufe der Leitfähigkeiten erfasst.

Die Dialysemaschine 2 sendet die Daten der erfassten Messwerte von z.B. Druck, Temperatur und Durchfluss aller vier Leitungen über die Schnittstelle 38 zeitaktuell, sprich ohne Zeitverzug, das bedeutet dass lediglich eine physikalisch auftretende Signallaufzeit mit zugehöriger Signalverarbeitung, aber keine weitere (gewollte und vermeidbare) Verzögerung auftritt, und kabellos bzw. alternativ oder zusätzlich auch kabelgebunden an eine Empfangseinheit 40 (als Teil einer überwachungsvorrichtungsseitigen Schnittstelle) der Überwachungsvorrichtung 1. Die Empfangseinheit 40 leitet die erfassten und abgegriffenen Messwerte an eine zentrale Steuereinheit 42 weiter, welche die Messwerte selektiert und weiterverarbeitet. Die zentrale Steuereinheit 42 weist dabei einen Erfassungsspeicher 44, in welcher ein Ist-Verlauf 46 aufgezeichnet wird, sowie einen Speicher 48, in welchem Soll-Verläufe 50 der jeweiligen Messwerte gespeichert sind, auf. Neben den Soll-Verläufen 50 der jeweiligen Messwerte sind in dem Speicher 48 auch Fehler-Verläufe 52 für die entsprechenden Messwerte hinterlegt. Der Erfassungsspeicher 44 sowie der Speicher 48 können physisch auch in einem gemeinsamen, einzigen Speicherelement ausgebildet sein.

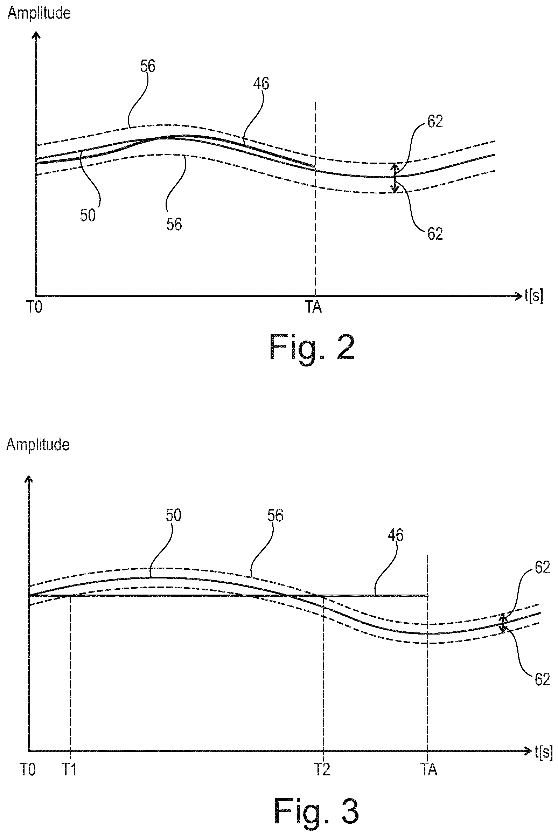

Eine Vergleichseinheit 54 der Überwachungsvorrichtung 1 vergleicht für jeden Messwert den Ist-Verlauf 46 mit dem Soll-Verlauf 50 sowie den Ist-Verlauf 46 mit dem oder den Fehler-Verläufen 52. Sollte der Ist-Verlauf 46 von dem Soll-Verlauf 50 über eine definierte Toleranz 56 hinaus abweichen, so bestimmt eine Bestimmungseinheit 58, dass ein Defekt der Dialysemaschine 2 vorliegt. Mit anderen Worten ermittelt die Bestimmungseinheit 58 also auf Basis des Ergebnisses des von der Vergleichseinheit 54 durchgeführten Vergleichs, ob ein Defekt vorliegt. Dies ist dann der Fall, wenn der Ist-Verlauf 46 von dem Soll-Verlauf 50 über eine definierte Toleranz 56 hinaus abweicht. Ebenfalls bestimmt die Bestimmungseinheit 58, dass ein Defekt vorliegt, wenn der Ist-Verlauf 46 innerhalb einer definierten Toleranz 56 einem Fehler-Verlauf 52 entspricht bzw. mit diesem übereinstimmt. Der Vergleich der jeweiligen Ist- mit den zugehörigen Soll- bzw. Fehlerverläufen durch die Vergleichseinheit 54 und die Durchführung der Bestimmung durch die Bestimmungseinheit 58, ob ein Defekt vorliegt, werden bei der Beschreibung der Figuren 2 und 3 detailliert erläutert.

Ist bestimmt, dass ein Defekt vorliegt, so werden über eine Ausgabeeinheit in Form eines Alarmgebers 60 ein akustisches, ein visuelles und/oder ein taktiles Alarmsignal ausgegeben. Ebenso wird über den Alarmgeber 60 ein Steuer-Befehl über eine Sendeeinheit 41 an die Schnittstelle 38 der Dialysemaschine 2 gesendet. Alternativ oder zusätzlich zu dem Steuer-Befehl kann die Überwachungsvorrichtung 1 über die Sendeeinheit 41 auch lediglich nur ein Fehlercode an die ECU 32 der Dialysemaschine 2 senden. Die Dialysemaschine 2 kann dann anhand des Fehlercodes unabhängig bestimmen, welche Maßnahmen dann entsprechend des Fehlercodes ergriffen werden. Somit agiert die Dialysemaschine 2 weiterhin "autonom" und die Steuerungsgewalt liegt allein bei der Dialysemaschine 2. Der Steuer-Befehl kann vorzugsweise die Anweisung an die ECU 32 der Dialysemaschine 2 geben, die Blutpumpe 18 und die Dialysierflüssigkeitspumpe 30 anzuhalten sowie die elektromagnetische Schlauchklemme 36 und das Ventil 37 vollständig zu schließen. Auch kann beispielsweise, wenn das Ventil 37 nicht vollständig schließt, obwohl dieses beispielsweise für einen Wechsel der Dialysierflüssigkeit geschlossen sein sollte, über die Sensoren 34 und über letztlich die Überwachungsvorrichtung bestimmt werden, dass ein Defekt vorliegt. Hiernach wird sofort die Schlauchklemme 36 geschlossen. Somit wird eine Gefährdung des Patienten bei einem detektierten Defekt direkt und unmittelbar unterbunden.

Figur 2 zeigt zur Erklärung der genauen Funktionsweise des Vergleichens und des Bestimmens durch die Vergleichseinheit 54 bzw. die Bestimmungseinheit 58 anschaulich einen Graphen mit beispielhaften Verläufen. Auf der Abszissenachse ist die Zeit (für den Verlauf) und auf der Ordinatenachse eine Amplitude bzw. ein Absolutwert des Messwertes der Verläufe aufgetragen. In dem Speicher 48 der Überwachungsvorrichtung 1 ist der Soll-Verlauf 50 in Form einer Sinuskurve (der Anschauung halber) hinterlegt. Als Kriterium für die Toleranz 56 ist eine absolute Messwertabweichung mit einem konstanten Abweichungswert 62 gewählt, welcher sowohl in die negative als auch die positive Richtung (der Amplitude) gilt. Somit ergibt sich um den Soll-Verlauf 50 herum eine Art Korridor/Schlauch/Band als definierte Toleranz 56 bzw. als Vertrauensbereich.

Der Ist-Verlauf 46 ist ebenfalls in Figur 2 eingezeichnet. Der Ist-Verlauf 46 ist ab dem Zeitpunkt T0 aufgenommen (einem Start der Aufzeichnung der Messwerte oder einem Start der Behandlung beispielsweise) und ist bis zu einer jetzigen, aktuellen Zeit TA aufgezeichnet und in dem Erfassungsspeicher 44 gespeichert. Wie aus Fig. 2 ersichtlich, liegt der Ist-Verlauf 46 innerhalb der Toleranz 56 des Soll-Verlaufs 50 für den entsprechenden Messwert. Damit bestimmt die Überwachungsvorrichtung 1 nicht, dass ein Defekt vorliegt oder in anderen Worten ausgedrückt, die Überwachungsvorrichtung 1 bestimmt, dass aktuell kein Defekt vorliegt.

An dieser Stelle sei angemerkt, dass der in Figur 2 dargestellte Graph für genau einen Messwert (von natürlich einer Leitung) gilt. Beispielsweise ist in Fig. 2 ein Ist-Verlauf 46 des Drucks der venösen Blutleitung 14 dargestellt. Auf die gleiche Weise kann natürlich auch ein Messwert wie eine Temperatur der Dialysierflüssigkeitszuleitung 24 herangezogen werden.

Figur 3 zeigt einen Ist-Verlauf 46, bei dem der Ist-Verlauf 46 konstant sowie abschnittsweise auch außerhalb der Toleranz 56 des Soll-Verlaufs 50 verläuft. Hierbei bestimmt die Überwachungsvorrichtung 1 zu einem ersten Zeitpunkt T1, bei der der Ist-Verlauf 46 die Toleranz 56 verlässt, oder, falls eine gewisse Zeit des Ist-Verlaufs 46 außerhalb der Toleranz 56 gewährt werden würde, spätestens zu einem zweiten Zeitpunkt T2, dass ein Defekt der Dialysemaschine 2 vorliegt. Die Behandlung wird gestoppt, die Pumpen 18, 30 angehalten und die Schlauchklemme 36 geschlossen.

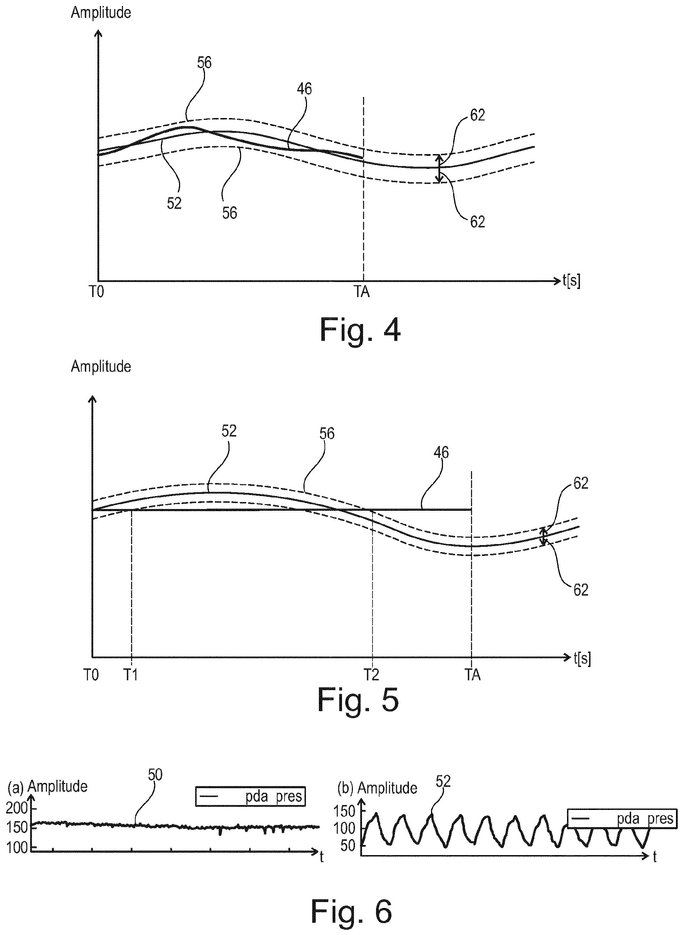

Figur 4 zeigt, ähnlich zu dem Vergleich aus Fig. 2, eine Gegenüberstellung des Ist-Verlaufs 46 mit dem Fehler-Verlauf 52. Die Überwachungsvorrichtung 1 vergleicht neben dem Soll-Verlauf 50 nämlich ebenso auch den Fehler-Verlauf 52 mit gleicher Vorgehensweise, allerdings mit dem Unterschied, dass, wenn der Ist-Verlauf 46 innerhalb der Toleranz 56 des Fehler-Verlaufs 52 liegt, die Bestimmungseinheit 58 bzw. die Überwachungsvorrichtung 1 nun bestimmt, dass tatsächlich ein Defekt vorliegt.

Figur 5 zeigt, ähnlich zu dem Vergleich aus Fig. 3, eine Gegenüberstellung des Ist-Verlaufs 46 mit dem Fehler-Verlauf 52, bei dem der Ist-Verlauf 46 nicht dem Fehler-Verlauf 52 folgt. Es wird bestimmt, dass (bis auf die Bereiche um die Zeitpunkte T1 und T2) der Ist-Verlauf 46 nicht dem Fehler-Verlauf 52 entspricht und damit kein Defekt vorliegt. Die Durchgänge des Ist-Verlaufs 46 in den Bereich des Korridors/Schlauchs (Toleranz 56) um den Fehler-Verlauf 52 müssen natürlich ausgewertet werden und es muss definiert werden, dass diese Abweichungen außerhalb der Toleranz liegen.

Wichtig ist, dass für jeden einzigen, einzelnen Messwert, wie beispielsweise der Temperatur, ein separater Soll-Verlauf 50 bzw. ein oder mehrere Fehler-Verläufe 52 zu genau diesem Messwert zu genau dieser Leitung in dem Speicher 48 hinterlegt sind. Gegebenenfalls kann man für jeden einzelnen Messwert sogar auch mehrere Soll-Verläufe 50 für beispielsweise unterschiedliche Therapiearten oder Modi der extrakorporalen Blutbehandlungsvorrichtung hinterlegen.

Figur 6 zeigt einen Graphen, wobei im linken Teil der Figur 6 zur Anschauung ein beispielhafter Druckverlauf (PDA - Drucksensor Dialysierflüssigkeit Auslauf) bei einer normalen Therapie gemäß einem Soll-Verlauf 50 gezeigt ist, wohingegen im rechten Teil der Figur 6 als Messwert ein Druckverlauf nach einem Fehler-Verlauf 52 zeigt, bei dem ein Defekt vorliegt und eine Therapie des Patienten gestört ist. Der Fehler-Verlauf 52 stellt eine Therapie mit einem defekten Bilanzkammerventil dar, wobei das Bilanzkammerventil nicht korrekt schließt. Der Graph bzw. der Druckverlauf ist infolge dessen in der Form eines Sägezahnprofils ausgebildet.

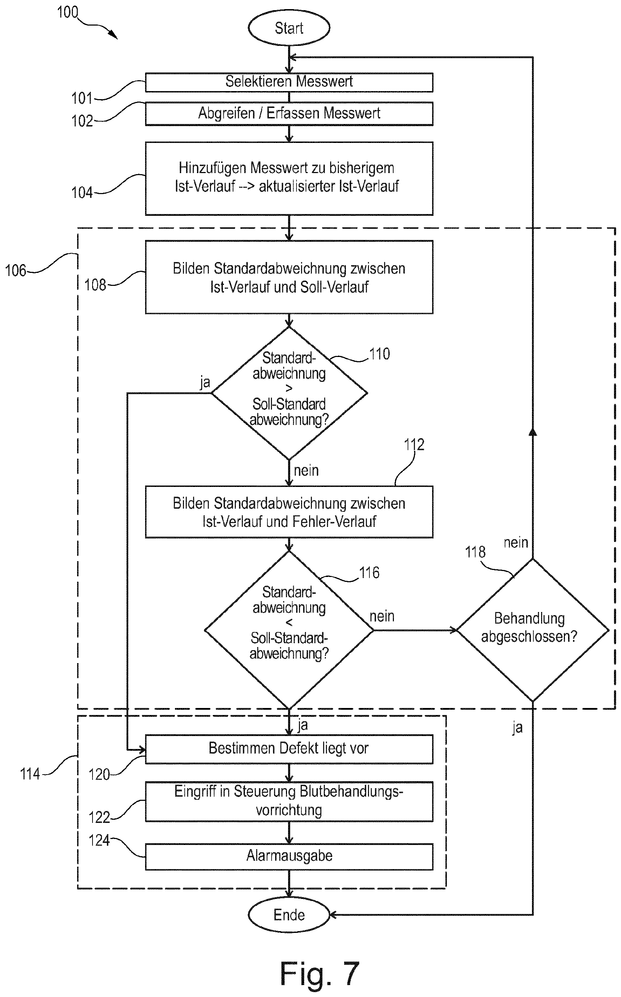

Figur 7 zeigt ein Verfahren 100 einer Variante zur Überwachung einer extrakorporalen Blutbehandlungsvorrichtung, wie etwa einer Dialysemaschine 2 (siehe Fig. 1), mit einem extrakorporalen Blutkreislauf, der eine arterielle Blutleitung 12 mit einem arteriellen Patientenanschluss 13 und/oder zumindest eine venöse Blutleitung 14 mit einem venösen Patientenanschluss 16 aufweist, und einem Dialysierflüssigkeitssystem 20, wobei während des Betriebs der extrakorporalen Blutbehandlungsvorrichtung ein erfasster Messwert durch die Überwachungsvorrichtung abgegriffen wird. Nach dem Start des Verfahrens 100 folgt in Schritt 101 ein Selektieren und in Schritt 102 ein kontinuierliches Abgreifen und damit Erfassen eines Messwertes. Das gezeigte Verfahren wird für einen Messwert, beispielsweise den Druck der venösen Blutleitung 14, beschrieben. Natürlich kann nicht nur ein Messwert, sondern es können auch mehrere Messwerte zu beispielsweise Druck, Temperatur und Flussrate von unterschiedlichen Abschnitten der Leitungen der Blutbehandlungsvorrichtung abgegriffen werden. Für jeden einzelnen Messwert wird dann das Verfahren 100 durchgeführt.

Nach dem Schritt des Abgreifens 102 schreitet das Verfahren 100 zum Schritt Hinzufügen 104 des erfassten Messwertes zum bisherigen Ist-Verlauf voran. Hierdurch wird ein bis zu der Zeit erfasster Ist-Verlauf um den zeitlich aktuellen Wert des Messwerts ergänzt, so dass kontinuierlich und zeitaktuell der aktualisierte Ist-Verlauf dem Verfahren 100 zur Verfügung steht. Nach dem Schritt Hinzufügen 104 wird im nachfolgenden Block Vergleichen 106 der Ist-Verlauf mit einem Soll-Verlauf und der Ist-Verlauf mit einem Fehler-Verlauf verglichen. Konkret schreitet der Prozess zu einem Schritt Bilden der Standardabweichung Soll-Verlauf 108 voran, bei dem eine Standardabweichung zwischen dem Ist-Verlauf und dem Soll-Verlauf gebildet wird. Im speziellen wird in diesem Schritt eine Standardabweichung der einzelnen kontinuierlich erfassten Messwerte von dem Zeitpunkt T0 bis zu dem Zeitpunkt TA (siehe auch Fig. 2 und 3) gebildet. Anstelle des Zeitpunkts T0 ist es auch vorstellbar einen späteren Zeitpunkt zu wählen, um nur einen bestimmten zurückliegenden Zeitabschnitt zu analysieren und in diesem Zeitabschnitt die Standardabweichung zwischen dem Ist-Verlauf und dem Soll-Verlauf zu bilden.

Als Ergebnis der Bildung der Standardabweichung in Schritt 108 liegt ein absoluter Wert vor, der in einer Bedingung 110 mit einem hinterlegten Soll-Wert bzw. einer hinterlegten Soll-Standardabweichung verglichen wird. Ist der Wert der Standardabweichung geringer als die Soll-Standardabweichung (Nein), und liegt damit der Ist-Verlauf innerhalb der Toleranz des Soll-Verlaufs, so schreitet das Verfahren zu einem Schritt Bilden der Standardabweichung Fehler-Verlauf 112 weiter. Sollte jedoch bei der Bedingung 110 die berechnete Standardabweichung größer als die Soll-Standardabweichung sein (Ja), liegt der Ist-Verlauf außerhalb der Toleranz des Soll-Verlaufs und das Verfahren schreitet zu einem Block Defektbestimmung 114 voran.

In dem Schritt Bilden der Standardabweichung Fehler-Verlauf 112 wird, im Wesentlichen gleich zu dem Schritt Bilden der Standardabweichung Soll-Verlauf 108, die Standardabweichung zwischen dem Ist-Verlauf und einem hinterlegten Fehler-Verlauf durchgeführt. Auch hier kann anstelle der Zeitspanne T0 bis TA eine beliebige Zeitspanne T1 oder T2 bis TA verwendet werden.

In einer nachfolgenden Bedingung 116 wird überprüft, ob die berechnete Standardabweichung geringer als eine Soll-Standardabweichung ist. Diese Soll-Standardabweichung der Bedingung 116 kann sich je nach Definition ggf. von der Soll-Standardabweichung des Schritts 108 unterscheiden. Beide Soll-Standardabweichungen sollen sein, um eine zentral zu steuernde Variable zu haben. Ist die berechnete Standardabweichung geringer als die Soll-Standardabweichung (Ja), so ist durch den Vergleich herausgekommen, dass die Ist-Kurve innerhalb der Toleranz der Fehler-Kurve liegt. Das Verfahren schreitet hiernach, wie auch bei der Bedingung 110, zu dem Block Defektbestimmung 114 voran. Ist die Standardabweichung jedoch größer als die Soll-Standardabweichung, so geht das Verfahren zu einer Bedingung 118 weiter, in der geprüft wird, ob die Behandlung abgeschlossen ist. An dieser Stelle sei angemerkt, dass natürlich auch mehrere Fehler-Verläufe 52 zu genau einem Messwert hinterlegt sein können. Sollte dies der Fall sein, so werden der Schritt 112 und die Bedingung 116 erneut für den jeden weiteren vorhandenen Fehler-Verlauf durchlaufen, so dass erst nach Prüfung aller Fehler-Verläufe des Messwertes das Verfahren zu Bedingung 118 oder dem Block Defektbestimmung 114 weitergeht.

In der Bedingung 118 wird der Status der Behandlung geprüft. Ist die Behandlung noch nicht abgeschlossen (Nein), so wird eine weitere Schleife dem Verfahren angefügt, indem das Verfahren zu dem Schritt des Abgreifens 102 geleitet wird. Ist hingegen die Behandlung des Patienten abgeschlossen (Nein), so entfällt ebenso ein Bedarf einer Überwachung der Blutbehandlungsvorrichtung und das Verfahren endet.

Der Block Defektbestimmung 114 beinhaltet den Schritt Bestimmen dass Defekt vorliegt 120, den Schritt Eingriff in Steuerung der Blutbehandlungsvorrichtung 122 sowie den Schritt der Alarmausgabe 124. In dem Schritt Eingriff in die Steuerung der Blutbehandlungsvorrichtung 122 wird durch das Verfahren, beispielsweise über einen gesendeten Steuer-Befehl und/oder einen entsprechenden Fehlercode an die Blutbehandlungsvorrichtung, die Blutbehandlungsvorrichtung gestoppt, die Pumpen angehalten und insbesondere ein Ventil bzw. eine Schlauchklemme der venösen Blutleitung und ein Ventil der Dialysierflüssigkeitszuleitung 24 geschlossen. Der Schritt der Alarmausgabe 124 kann eine optische Alarmausgabe, beispielsweise auf einem Display der Blutbehandlungsvorrichtung, einem blinkenden Licht, einer akustischen Alarmgabe in Form eines Piepsens und einer taktilen Alarmgabe wie einer Vibration erfolgen. Nach dem Block der Defektbestimmung 114 stoppt das Verfahren.

### Bezugszeichenliste

- 1: Überwachungsvorrichtung
- 2: Dialysemaschine
- 4: Dialysator
- 6: Semipermeable Membran
- 8: Erste Kammer
- 10: Zweite Kammer
- 12: Arterielle Blutleitung
- 13: Punktionskanüle für arteriellen Patientenanschluss
- 14: Venöse Blutleitung
- 16: Punktionskanüle für venösen Patientenanschluss
- 18: Blutpumpe
- 20: Dialysierflüssigkeitssystem
- 22: Dialysierflüssigkeitsquelle
- 24: Dialysierflüssigkeitszuleitung
- 26: Dialysierflüssigkeitsableitung
- 28: Dialysierflüssigkeitsabfluss
- 30: Dialysierflüssigkeitspumpe
- 32: elektronische Steuereinheit / ECU
- 34: Sensor
- 36: Schlauchklemme
- 37: Ventil
- 38: Schnittstelle
- 40: Empfangseinheit
- 41: Sendeeinheit
- 42: Zentrale Steuereinheit
- 44: Erfassungsspeicher
- 46: Ist-Verlauf
- 48: Speicher
- 50: Soll-Verlauf
- 52: Fehler-Verlauf
- 54: Vergleichseinheit
- 56: Toleranz
- 58: Bestimmungseinheit
- 60: Alarmgeber
- 62: Abweichungswert

- T0: Startzeit
- T1: erster Zeitpunkt
- T2: zweiter Zeitpunkt
- TA: aktuelle Zeit

- 100: Verfahren
- 101: Schritt Selektieren
- 102: Schritt Abgreifen
- 104: Schritt Hinzufügen Messwert
- 106: Block Vergleichen
- 108: Schritt Bilden Standardabweichung
- 110: Vergleichsbedingung
- 112: Schritt Bilden Standardabweichung
- 114: Block Defektbestimmung
- 116: Vergleichsbedingung
- 118: Bedingung Behandlungsabschluss
- 120: Schritt Bestimmen Defekt
- 122: Schritt Eingriff in Steuerung
- 124: Schritt Alarmausgabe

## Patentansprüche

1. Überwachungsvorrichtung (1) zur Überwachung einer extrakorporalen Blutbehandlungsvorrichtung (2), wie etwa einer Dialysemaschine, mit einem extrakorporalen Blutkreislauf, der eine arterielle Blutleitung (12) mit einem arteriellen Patientenanschluss (13) und/oder zumindest eine venöse Blutleitung (14) mit einem venösen Patientenanschluss (16) aufweist, und einem Dialysierflüssigkeitssystem (20), das eine Dialysierflüssigkeitszuleitung (24) und eine Dialysierflüssigkeitsableitung (26) aufweist,
**dadurch gekennzeichnet, dass** in einem Speicher (48) der Überwachungsvorrichtung (1) neben dem Soll-Verlauf (50) des Messwertes ein oder mehrere Fehler-Verläufe (52) des Messwertes hinterlegt sind und
die Überwachungsvorrichtung (1) konfiguriert ist, einen durch und während des Betriebs der extrakorporalen Blutbehandlungsvorrichtung (2) auftretenden und erfassten Messwert, welcher zur Überwachung der Blutbehandlungsvorrichtung (2) geeignet ist, zu selektieren und abzugreifen, einen zeitlichen Ist-Verlauf (46) des erfassten Messwertes mit einem in dem Speicher (48) der Überwachungsvorrichtung (1) hinterlegten Soll-Verlauf (50) des zugehörigen Messwertes und mit zumindest einem des ein oder mehreren Fehler-Verläufen (42) des zugehörigen Messwerts zu vergleichen und zu bestimmen, dass ein Defekt vorliegt, wenn zumindest abschnittsweise der Ist-Verlauf (46) des erfassten Messwertes von dem Soll-Verlauf (50) des hinterlegten Messwertes über eine erste definierte Toleranz (56) hinaus abweicht und wenn zumindest abschnittsweise der Ist-Verlauf (46) des erfassten Messwertes innerhalb einer zweiten definierten Toleranz (56) mit dem hinterlegten Fehler-Verlauf (52) des Messwertes übereinstimmt.

2. Überwachungsvorrichtung (1) nach Anspruch 1, **dadurch gekennzeichnet, dass** die Überwachungsvorrichtung (1) die Messwerte während eines Betriebs der extrakorporalen Blutbehandlungsvorrichtung (2) ohne Zeitverzug zeitaktuell erfasst.

3. Überwachungsvorrichtung (1) nach einem der vorangegangenen Ansprüche, **dadurch gekennzeichnet, dass** die Überwachungsvorrichtung (1) eine Spannung und/oder einen Strom und/oder eine Drehzahl einer in der extrakorporalen Blutbehandlungsvorrichtung (2) in dem extrakorporalen Blutkreislauf zum Fördern von Blut und/oder in der Dialysierflüssigkeitssystem (20) zum Fördern von Dialysierflüssigkeit angeordneten Pumpe (18; 30) als erfassten Messwert abgreift.

4. Überwachungsvorrichtung (1) nach einem der vorangegangenen Ansprüche, **dadurch gekennzeichnet, dass** die Überwachungsvorrichtung (1) einen Druck und/oder eine Temperatur und/oder einen Durchfluss und/oder eine Leitfähigkeit und/oder eine Membranposition als erfassten Messwert abgreift.

5. Überwachungsvorrichtung (1) nach einem der vorangegangenen Ansprüche, **dadurch gekennzeichnet, dass** die Überwachungsvorrichtung (1) nach einem Startsignal für eine vorbestimmte Detektions-Zeit den Ist-Verlauf (46) des/der jeweiligen Messwerte detektiert und diesen detektierten Ist-Verlauf (46) jeweils als den in dem Speicher hinterlegten Soll-Verlauf (50) des entsprechenden Messwerts festsetzt.

6. Überwachungsvorrichtung (1) nach einem der vorangegangenen Ansprüche, **dadurch gekennzeichnet, dass** als Kriterium für die erste und/ oder zweite definierte Toleranz (56) eine Steigung und/oder eine absolute Messwertabweichung und/oder eine Standardabweichung zwischen dem Ist-Verlauf (46) und dem Soll-Verlauf (50) und/oder zwischen dem Ist-Verlauf (46) und dem Fehler-Verlauf (52) herangezogen werden.

7. Überwachungsvorrichtung (1) nach Anspruch 6, **dadurch gekennzeichnet, dass** als Kriterium für die erste und/ oder zweite definierte Toleranz (56) die Steigung und die absolute Messwertabweichung und die Standardabweichung herangezogen werden.

8. Überwachungsvorrichtung (1) nach einem der vorangegangenen Ansprüche, **dadurch gekennzeichnet, dass** als Kriterium für die definierte Toleranz (56) ein Minimum und/oder ein Maximum über einen definierten Zeitraum des Ist-Verlaufs des erfassten Messwerts gegenüber dem hinterlegten Soll-Verlauf (50) und/oder dem hinterlegten Fehler-Verlauf (52) des Messwerts herangezogen werden.

9. Überwachungsvorrichtung (1) nach einem der vorangegangenen Ansprüche, **dadurch gekennzeichnet, dass** zumindest zwei Messwerte erfasst und abgegriffen werden und die Überwachungsvorrichtung ein Verhalten und/oder eine Korrelation der Messwerte zueinander bei dem Vergleich der Verläufe und der Bestimmung, dass ein Defekt vorliegt, heranzieht.

10. Überwachungsvorrichtung (1) nach einem der vorangegangenen Ansprüche, **dadurch gekennzeichnet, dass** bei Bestimmung eines Defekts durch die Überwachungsvorrichtung (1) ein akustisches, visuelles und/oder taktiles Signal durch eine Ausgabeeinheit (60) ausgegeben wird und/oder ein entsprechender Fehlercode an die extrakorporale Blutbehandlungsvorrichtung (2) ausgegeben wird.

11. Verfahren (100) zur Überwachung einer extrakorporalen Blutbehandlungsvorrichtung, wie etwa einer Dialysemaschine (2), mit einem extrakorporalen Blutkreislauf, der eine arterielle Blutleitung (12) mit einem arteriellen Patientenanschluss (13) und/oder zumindest eine venöse Blutleitung (14) mit einem venösen Patientenanschluss (16) aufweist, und einem Dialysierflüssigkeitssystem (20), das eine Dialysierflüssigkeitszuleitung (24) und eine Dialysierflüssigkeitsableitung (26) aufweist, wobei während des Betriebs der extrakorporalen Blutbehandlungsvorrichtung (2) ein erfasster Messwert abgegriffen wird, **gekennzeichnet durch** die Schritte:
Selektieren (101) des Messwerts, welcher zur Überwachung der Blutbehandlungsvorrichtung (2) und einer Komponente der Blutbehandlungsvorrichtung (2) geeignet ist;
Kontinuierliches Abgreifen (102) und damit Erfassen des Messwerts;
Vergleichen (106) eines Ist-Verlaufs (46) des erfassten Messwertes mit einem Soll-Verlauf (50) und mit zumindest einem Fehler-Verlauf (52) des zugehörigen Messwertes; und
Bestimmen (114), dass ein Defekt vorliegt, wenn zumindest abschnittsweise der Ist-Verlauf (46) des erfassten Messwertes von dem Soll-Verlauf (50) über eine erste definierte Toleranz (56) hinaus abweicht und wenn der Ist-Verlauf (46) des erfassten Messwertes innerhalb einer zweiten definierten Toleranz (56) mit dem Fehler-Verlauf (52) übereinstimmt.

12. Verfahren zur Überwachung nach Anspruch 11, **dadurch gekennzeichnet, dass** der Schritt Vergleichen (106) folgende Schritte umfasst:
Bilden der Standardabweichung (108; 112) zwischen dem Ist-Verlauf (46) des Messwertes mit dem Soll-Verlauf (50) und/oder dem Fehler-Verlauf (52) des Messwertes;
Vergleichen (110; 116) des Werts der gebildeten Standardabweichung mit einer hinterlegten Soll-Standardabweichung.

## Claims

1. A monitoring device (1) for monitoring an extracorporeal blood treatment device (2), such as a dialysis machine, comprising an extracorporeal blood circuit which has an arterial blood line (12) with an arterial patient port (13) and/or at least one venous blood line (14) with a venous patient port (16), and a dialysis fluid system (20) which has a dialysis fluid supply line (24) and a dialysis fluid drain line (26),
**characterized in that** one or more error courses (52) of a measured value are stored in a memory (48) of the monitoring device (1) in addition to a target course (50) of the measured value, and
the monitoring device (1) is configured to select and sense the measured value occurring and detected by and during operation of the extracorporeal blood treatment device (2) and which is suitable for monitoring the blood treatment device (2), to compare a time-related actual course (46) of the detected measured value with the target course (50) of the associated measured value stored in the memory (48) of the monitoring device (1) and with at least one of the one or more error courses (42) of the associated measured value, and to determine that there is a defect if, at least in sections, the actual course (46) of the detected measured value deviates from the target course (50) of the stored measured value by more than a first defined tolerance (56), and if, at least in sections, the actual course (46) of the detected measured value coincides with the stored error course (52) of the measured value within a second defined tolerance (56).

2. The monitoring device (1) according to claim 1, **characterized in that** the monitoring device (1) detects the measured values during operation of the extracorporeal blood treatment device (2) in real time without any time delay.

3. The monitoring device (1) according to any of the preceding claims, **characterized in that** the monitoring device (1) senses a voltage and/or a current and/or a rotational speed of a pump (18; 30) arranged in the extracorporeal blood treatment device (2) in the extracorporeal blood circuit for conveying blood and/or in the dialysis fluid system (20) for conveying dialysis fluid, as the detected measured value.

4. The monitoring device (1) according to any of the preceding claims, **characterized in that** the monitoring device (1) senses a pressure and/or a temperature and/or a flow and/or a conductivity and/or a membrane position as the detected measured value.

5. The monitoring device (1) according to any of the preceding claims, **characterized in that** the monitoring device (1) detects the actual course (46) of the respective measured value(s) after a start signal for a predetermined detection time and defines this detected actual course (46) in each case as the target course (50) of the corresponding measured value stored in the memory.

6. The monitoring device (1) according to any of the preceding claims, **characterized in that** a gradient and/or an absolute measured value deviation and/or a standard deviation between the actual course (46) and the target course (50) and/or between the actual course (46) and the error course (52) are used as criterion for the first and/or second defined tolerance (56).

7. Monitoring device (1) according to claim 6, **characterized in that** the gradient, the absolute measured value deviation, and the standard deviation are used as criteria for the first and/or second defined tolerance (56).

8. The monitoring device (1) according to any of the preceding claims, **characterized in that** a minimum and/or a maximum over a defined time span of the actual course of the detected measured value with respect to the stored target course (50) and/or the stored error course (52) of the measured value are used as criterion for the defined tolerance (56).

9. The monitoring device (1) according to any of the preceding claims, **characterized in that** at least two measured values are detected and sensed and the monitoring device uses a behavior and/or a correlation of the at least two measured values between them when comparing the courses and determining whether there is a defect.

10. The monitoring device (1) according to any of the preceding claims, **characterized in that,** when a defect is determined by the monitoring device (1), an acoustic, visual and/or tactile signal is output by an output unit (60) and/or a corresponding error code is output to the extracorporeal blood treatment device (2).

11. A method (100) for monitoring an extracorporeal blood treatment device, such as a dialysis machine (2), comprising an extracorporeal blood circuit which has an arterial blood line (12) with an arterial patient port (13) and/or at least one venous blood line (14) with a venous patient port (16), and a dialysis fluid system (20) comprising a dialysis fluid supply line (24) and a dialysis fluid drain line (26), wherein during operation of the extracorporeal blood treatment device (2) a detected measured value is sensed, **characterized by** the steps:
selecting (101) the measured value which is suitable for monitoring the blood treatment device (2) and a component of the blood treatment device (2);
continuously sensing (102) and thereby detecting the measured value;
comparing (106) an actual course (46) of the detected measured value with a target course (50) and with at least one error course (52) of the associated measured value; and
determining (114) that there is a defect if, at least in sections, the actual course (46) of the detected measured value deviates from the target course (50) by more than a first defined tolerance (56) and if the actual course (46) of the detected measured value coincides with the error course (52) within a second defined tolerance (56).

12. The method for monitoring according to claim 11, **characterized in that** the step (106) of comparing comprises the following steps:
forming the standard deviation (108; 112) between the actual course (46) of the measured value with the target course (50) and/or the error course (52) of the measured value;
comparing (110; 116) the value of the formed standard deviation with a stored target standard deviation.

## Revendications

1. Dispositif de surveillance (1) pour la surveillance d'un dispositif de traitement du sang (2) extracorporel, comme une machine de dialyse, avec un circuit sanguin extracorporel qui présente une conduite sanguine (12) artérielle avec un raccord patient (13) artériel et/ou au moins une conduite sanguine (14) veineuse avec un raccord patient (16) veineux et un système de liquide de dialyse (20) qui présente une alimentation en liquide de dialyse (24) et une évacuation de liquide de dialyse (26),
**caractérisé en ce qu'** une ou plusieurs courbes d'erreur (52) de la valeur de mesure sont enregistrées dans une mémoire (48) du dispositif de surveillance (1), en plus de la courbe de consigne (50) de la valeur de mesure et
le dispositif de surveillance (1) est configuré afin de sélectionner et de récupérer une valeur de mesure détectée et survenant par et pendant le fonctionnement du dispositif de traitement du sang (2) extracorporel qui est appropriée pour la surveillance du dispositif de traitement du sang (2), de comparer une courbe réelle temporelle (46) de la valeur de mesure détectée avec une courbe de consigne (50) de la valeur de mesure afférente enregistrée dans la mémoire (48) du dispositif de surveillance (1) et avec au moins une des une ou plusieurs courbes d'erreur (42) de la valeur de mesure afférente et de déterminer qu'un défaut existe lorsque la courbe réelle (46) de la valeur de mesure détectée diverge de la courbe de consigne (50) de la valeur de mesure enregistrée au moins par sections au-delà d'une première tolérance définie (56) et lorsque la courbe réelle (46) de la valeur de mesure détectée coïncide au moins par sections avec la courbe d'erreur enregistrée (52) de la valeur de mesure dans une seconde tolérance définie (56).

2. Dispositif de surveillance (1) selon la revendication 1, **caractérisé en ce que** le dispositif de surveillance (1) détecte en temps réel les valeurs de mesure pendant un fonctionnement du dispositif de traitement extracorporel du sang (2) sans délai.

3. Dispositif de surveillance (1) selon l'une quelconque des revendications précédentes, **caractérisé en ce que** le dispositif de surveillance (1) récupère une tension et/ou un courant et/ou une vitesse de rotation d'une pompe (18 ; 30) agencée dans le dispositif de traitement du sang (2) extracorporel dans le circuit sanguin extracorporel pour le transport de sang et/ou dans le système de liquide de dialyse (20) pour le transport de liquide de dialyse (18 ; 30) comme valeur de mesure détectée.

4. Dispositif de surveillance (1) selon l'une quelconque des revendications précédentes, **caractérisé en ce que** le dispositif de surveillance (1) récupère une pression et/ou une température et/ou un débit et/ou une conductivité et/ou une position de membrane comme valeur de mesure détectée.

5. Dispositif de surveillance (1) selon l'une quelconque des revendications précédentes, **caractérisé en ce que** le dispositif de surveillance (1) détecte, après un signal de départ pour un temps de détection prédéterminé, la courbe réelle (46) de la/des valeur(s) de mesure respective(s) et définit cette courbe réelle détectée (46) respectivement comme la courbe de consigne (50) de la valeur de mesure correspondante enregistrée dans la mémoire.

6. Dispositif de surveillance (1) selon l'une quelconque des revendications précédentes, **caractérisé en ce qu'** un pas et/ou un écart par rapport à la valeur de mesure absolue et/ou un écart standard entre la courbe réelle (46) et la courbe de consigne (50) et/ou entre la courbe réelle (46) et la courbe d'erreur (52) sont utilisés comme critère pour la première et/ou seconde tolérance (56) définie.

7. Dispositif de surveillance (1) selon la revendication 6, **caractérisé en ce que**le pas et l'écart par rapport à la valeur de mesure absolue et l'écart standard sont utilisés comme critère pour la première et/ou seconde tolérance (56) définie.

8. Dispositif de surveillance (1) selon l'une quelconque des revendications précédentes, **caractérisé en ce qu'**un minimum et/ou un maximum sont utilisés sur une période définie de la courbe réelle de la valeur de mesure détectée par rapport à la courbe de consigne enregistrée (50) et/ou à la courbe d'erreur enregistrée (52) de la valeur de mesure comme critère pour la tolérance (56) définie.

9. Dispositif de surveillance (1) selon l'une quelconque des revendications précédentes, **caractérisé en ce qu'**au moins deux valeurs de mesure sont détectées et récupérées, et le dispositif de surveillance utilise un comportement et/ou une corrélation des valeurs de mesure l'une avec l'autre lors de la comparaison des courbes et de la détermination qu'un défaut existe.

10. Dispositif de surveillance (1) selon l'une quelconque des revendications précédentes, **caractérisé en ce que,** lors de la détermination d'un défaut par le dispositif de surveillance (1), un signal acoustique, visuel et/ou tactile est émis par une unité d'émission (60) et/ou un code d'erreur correspondant est transmis au dispositif de traitement du sang (2) extracorporel.

11. Procédé (100) de surveillance d'un dispositif de traitement extracorporel du sang, comme une machine de dialyse (2), avec un circuit sanguin extracorporel qui présente une conduite sanguine (12) artérielle avec un raccord patient (13) artériel et/ou au moins une conduite sanguine (14) veineuse avec un raccord patient (16) veineux et un système de liquide de dialyse (20) qui présente une alimentation en liquide de dialyse (24) et une évacuation de liquide de dialyse (26), dans lequel une valeur de mesure détectée est récupérée pendant le fonctionnement du dispositif de traitement extracorporel du sang (2),**caractérisé par** les étapes suivantes :
la sélection (101) de la valeur de mesure qui est appropriée pour la surveillance du dispositif de traitement du sang (2) et d'un composant du dispositif de traitement du sang (2) ;
la récupération continue (102) et ainsi la détection de la valeur de mesure ;
la comparaison (106) d'une courbe réelle (46) de la valeur de mesure détectée avec une courbe de consigne (50) et avec au moins une courbe d'erreur (52) de la valeur de mesure afférente ; et
la détermination (114) qu'un défaut existe lorsque la courbe réelle (46) de la valeur de mesure détectée diverge de la courbe de consigne (50) au moins par sections au-delà d'une première tolérance (56) définie et lorsque la courbe réelle (46) de la valeur de mesure détectée coïncide avec la courbe d'erreur (52) dans une seconde tolérance définie (56).

12. Procédé de surveillance selon la revendication 11, **caractérisé en ce que** l'étape de comparaison (106) comprend les étapes suivantes :
la formation de l'écart standard (108 ; 112) entre la courbe réelle (46) de la valeur de mesure et la courbe de consigne (50) et/ou la courbe d'erreur (52) de la valeur de mesure ;
la comparaison (110 ; 116) de la valeur de l'écart standard formé avec un écart standard de consigne enregistré.
